# EUROPEAN PATENT SPECIFICATION

(11) **EP 3 747 289 B1**
(45) Date of publication and mention of the grant of the patent: **26.01.2022**
(21) Application number: 19178687.0
(22) Date of filing: 06.06.2019
(51) Int. Cl.: A24F 47/00, A61M 15/06, H05B 6/80

(54) **MICROWAVE HEATING UNIT AND METHOD**
MIKROWELLENHEIZAGGREGAT UND VERFAHREN
UNITÉ ET PROCÉDÉ DE CHAUFFAGE PAR MICRO-ONDES

(43) Date of publication of application: 09.12.2020
(73) Proprietor: NVX Labs GmbH, 6302 Zug (CH)
(72) Inventor: KÜHN, Silvio, 16348 Wandlitz (DE); DUMLER, Ralf, 1870 Monthey (CH); TORREÑO NÚÑEZ, Alberto, 6890 Lustenau (AT)
(74) Representative: Hoefer & Partner Patentanwälte mbB

(56) References cited:
- EP-A1- 1 639 862
- WO-A1-2007/007123
- CN-A- 108 552 613
- CN-A- 108 777 893
- US-A1- 2011 139 773

## Description

The present invention refers to microwave heating units and methods which are in particular configured for heating an aerosol-forming sample due to microwave absorption by a material of the sample and in particular for thereby releasing by or from said sample upon heating said sample at least one aerosol and/or in a or as a inhalation, vaporizer and/or smoking product or device, in particular for medical and/or pulmonary drug delivery applications.

There are devices as consumer products known, for instance such as E-cigarettes or the like, which are configured in order to release an aerosol from a sample due to heating. However, these known devices suffer from incomplete, non-uniform and/or comparable slow heating of the sample. Accordingly, the required temperature and/or temperature distribution cannot be achieved for realizing a suitable heating process in order to serve all the consumers requirements.

US 2011 / 139 773 A1 discloses a microwave heater and a method of heating. The microwave heater includes a non-modal interplate microwave applicator and may include a nonresonant enclosure. The non-modal interplate microwave applicator is configured to receive therein a load to be heated by microwaves radiated from the non-modal interplate microwave applicator.

CN 108 552 613 A discloses an electronic cigarette for atomizing by microwave resonance, comprising a main shell, a battery, a controller and a microwave generator which are located in the main shell, and an accommodating chamber used for placing a microwave resonance atomizer, wherein a gear switch capable of performing microwave power strength adjustment, a master control switch, a display screen and a charging interface are arranged on the outer wall of the main shell, and an air inlet is formed in the bottom of the outerwall of the main shell; the microwave resonance atomizer comprises a tar storage unit, an atomizing unit, an upper cover plate, a suction nozzle and a base used for fixing the microwave resonance atomizer in the accommodating chamber; and the microwave resonance atomizer is enveloped by a detachable auxiliary shell.

CN 108 777 893 Adiscloses a microwave heating device for an electronic cigarette. The microwave heating device comprises a base, a microwave generator and a microwave incident cavity, a heating cavity is formed in the base, a microwave transmission sheet is arranged between the heating cavity and the microwave incident cavity and is fixedly connected to the top end of the microwave incident cavity, a microwave emitting port is formed in the microwave incident cavity, the bottom of the microwave emitting port is fixedly connected to the microwave generator, and a battery module is arranged at the bottom of the microwave generator.

It is an object underlying the present invention to provide alternative microwave heating units and methods which have improved heating and thus aerosol releasing capabilities.

The object underlying the present invention is achieved by a microwave heating unit according and by a microwave heating method according to the independent claims. Preferred embodiments are defined in the respective dependent claims.

According to a first aspect of the present invention a microwave and/or RF (radio frequency) heating unit for heating an aerosol-forming sample due to microwave and/or RF absorption by a material of the sample and in particular for thereby releasing by or from said sample upon heating said sample at least one aerosol is provided, in particular in a or as a inhalation, vaporizer and/or smoking product or device, in particular for pulmonary drug delivery.

The microwave and/or RF heating unit according to the present invention comprises:
(i) a sample holding and exposing unit configured to receive and hold a sample in a holding and exposing space and to expose the sample to a microwave radiation field within said holding and exposing space,
(ii) a microwave radiation and/or RF unit configured to supply the microwave and/or RF radiation field and/or an underlying microwave and/or RF radiation signal to said holding and exposing space,
(iii) an impedance matching unit configured to achieve impedance matching between the holding and exposing space and the microwave and/or RF radiation field and/or the underlying microwave and/or RF radiation signal and optionally
(iv) a free running oscillator which is configured to generate, and supply said microwave and/or RF radiation field and/or said underlying microwave and/or RF radiation signal;
wherein said sample holding and exposing unit comprises a sample holder defining and forming said holding and exposing space within an interior, in particular as a cavity, by means of first and second holding portions and/or first and second holding portions separated by a slit structure,and wherein the first and second holding portions simultaneously serve as first and second electrodes, respectively.

By these measures a comparable high degree of reliability of the heating process can be achieved, in particular in terms of rapidity, uniformity and/or completeness. This is in particular achieved by means of a contactless and/or non-the resistive heating process underline the invention's microwave heating unit and method. According to the invention, said sample holding and exposing unit comprises a sample holder defining and forming said holding and exposing space within an interior, in particular as a cavity, by means of first and second holding portions and/or first and second holding portions separated by a slit structure.

Advantageously the impedance matching unit and in particular an impedance matching network thereof is or are tunable in its matching properties concerning at least one of impedance, frequency and the aerosol-forming sample. By these measures the effectiveness and/or uniformity of the microwave heating process can be further improved.

The impedance matching unit and in particular an impedance matching network thereof may comprise at least one of
- electrodes for generating the microwave radiation field upon receipt of an underlying microwave radiation signal and/or which geometrically fit to the aerosol-forming sample, holding and exposing space and/or a or the cavity thereof,
- an output which contains waveguide structures, transmission lines and/or stubs,
- 1/8 λ to 1/4 λ wave guide matching elements, wherein λ denotes the wavelength of the underlying microwave radiation, and
- one or plural dielectric material portions,
by means of which the impedance matching unit is in particular tunable in its properties.

Further additionally or alternatively the microwave radiation unit may be or may comprise one or plural microwave radiation sources and/or microwave radiation signal sources, in particular in the form of solid-state microwave sources or and/or microwave radiation signal sources, respectively.

Further additionally or alternatively a connecting configuration regarding such one or plural microwave radiation sources and/or microwave radiation signal sources may be provided, set connecting configuration having a connector and/or a waveguide for providing connection to the respective source.

Said one or plural microwave radiation (signal) sources and/or solid-state microwave (signal) sources may be configured such that upon energization they are capable of generating and/or releasing said underlying microwave radiation field and/or said microwave radiation field signal.

Accordingly, a power source unit may be provided which is configured in order to energize the underlying microwave radiation unit and in particular the one or plural microwave radiation (signal) sources and which in particular comprises one or plural DC power sources.

Said microwave radiation unit and in particular a microwave radiation (signal) source thereof may be or may comprise a transistor amplifier.

The impedance matching unit and in particular an impedance matching network thereof may be or may comprise a microwave feedback port and/or a microwave signal feedback port which is connected to an input of the transistor amplifier in order to meet an underlying oscillation condition.

Under such circumstances it is of particular advantage if an additional smart phase and/or an amplitude correction device is or are connected in between the feedback port and the input of the transistor amplifier.

The microwave radiation generating and/or releasing unit and in particular a microwave source thereof may be or may comprise a transistor amplifier and in particular may have an input that is connected to an output of a low power signal source.

Under such circumstances the low power signal source may be or may comprise at least one of a phased locked loop (PLL), a voltage-controlled oscillator (VCO) and a direct digital synthesis (DDS).

A Microwave and/or RF heating unit according to the present invention may be configured in order to at least one of
- process an aerosol-forming sample which is formed as or comprises at least one of a liquid substance, a solid substance, a medical cannabis containing substance, a nicotine containing substance, a phyto-active substance, a botanical drug substance, a pharmaceutical active substance and a tobacco substance, and
- to release said at least one aerosol comprising at least one of a phyto-active substance, botanical drug substance and a pharmaceutical active ingredient.

In distinct concrete embodiments, the invention's microwave heating unit may be formed in or as at least one of an a drug delivery product and/or device, inhalation product and/or device, smoking product and/or device, a mobile product and/or device and/or a portable product and/or device.

According to a further alternative or additional preferred embodiment of the invention's microwave and/or RF heating unit the underlying free running oscillator at least one of
- is connected to the sample holding and exposing unit or is a part thereof,
- is, comprises and/or realizes an IQ modulator and/or the functionality of an IQ modulator which means the ability of controlling the phase and / or the gain and
- comprises within a feedback loop a or the transistor amplifier, an or the impedance matching unit and/or impedance matching network and a or the amplitude/phase correction device.

The operation and thus the oscillation process of the free running oscillator is initiated by the statistical and/or stochastic properties and in particular by noise of the overall electric circuit. Amplitude, phase and gain properties are defined by the electric properties of the further components and/or geometry and material portions of the sample, the sample holding and exposing means and in particular the sample holder with the holding and exposing space and in particular the cavity and the impedance matching unit and network, for instance the slit structure and any dielectric.

In general and in an initial stage of the oscillation process, all frequency components are contained according to its ratio within the noise spectrum. However and based on the further properties of the entire circuit and in particular of an employed face an amplitude correction device, the required signal can be obtained in amplified formed by and according feedback process.

In this regard, the phase and amplitude correction device is able to shift the overall phase response as well as the overall gain of the amplifier or amplifier chain and resonant impedance matching to meet the phase and amplitude condition at desired frequency to make the circuit to an oscillator. A slight tuning of frequency of oscillation is possible by change the phase part. With adjusting the overall gains amplitude, the second mayor oscillation condition is controllable and needs to meet at small signal condition a gain of at least one. It can be used for power and efficiency control.

Of course, the impedance matching unit and network and the properties are of importance, too:
In general a high electrical field at the given frequency is required to heat up the sample dielectrically. When using signals at low frequency, transformers and/or lumped elements like capacitors and inductors/coils building resonant circuits which can be used to match the generator output impedance to the high impedance behavior of the electrodes for dielectric heating of the sample. The electrical loss of the sample material given by its tan(δ) property does provide the real part of the impedance and causes heating of the sample when inserting electrical fields.

At higher frequencies it is more suitable to use transmission lines and waveguides to match electrodes and signal source. Which means increase the voltage amplitude of the source output. The electrodes of the holding structure have a slightly capacitive impedance cause of its geometry, e.g. in the order of magnitude of 100 fF. A transmission line - e.g. as a part of the impedance matching unit 30, namely 30a) with a length of about 1/8 λ to about 1/5 λ transforms it to lower capacitive impedance which is equivalent to higher capacitance. When connecting a coil or the short circuit with a short waveguide less the 1/10 λ at this point, the inductive and capacitive part compensate each other and build a resonance circuit. This type of impedance matching transforms the electrical losses of the sample into a suitable impedance range for microwave signal sources e.g. transistor output stages.

The present invention further relates to microwave and/or RF (radio frequency) heating method for heating an aerosol-forming sample due to microwave absorption by a material of said sample and in particular for thereby releasing by or from said sample upon heating said sample at least one aerosol, the at least one aerosol comprising at least one of a phyto-active substance and a pharmaceutical active ingredient, wherein a microwave heating unit according to any one of the preceding claims is used for heating said sample.

Different entities may be used and/or heated as a sample and/or as a part thereof, e.g.:
- an aerosol-forming substrate of a smoking article, in particular of a nicotine and/or non-nicotine containing smoking article,
- a tobacco-loaded solid-aerosol forming sample of a smoking sample, in particular of a nicotine and/or non-nicotine containing smoking sample,
- an aerosol-forming substrate with or of a nicotine and/or non-nicotine containing liquid,
- an aerosol-forming active pharmaceutical ingredient,
- an aerosol-forming phyto-active substance and in particular from raw plant material and/or from a plant material,
- an aerosol-forming delta-9-Tetrahydrocanabinol (THC) and/or cannabidiol (CBD) and/or cannabinoids containing plant material,
- an aerosol-forming botanical drug substance and/or a botanical drug product, and/or
- an aerosol-forming substrate having at least one of microwave sensitizers and/or absorbers of the group of substances which comprises functionalized polysilsesquioxanes, carbon nanotubes, graphite, graphene, activated carbon, activated charcoal, metal powder, semiconductor powder and combinations and mixtures thereof,
or any of their combinations.

In order to achieve a higher microwave efficiency, materials referred to herein as sensitizers may be a part of or may be mixed with the aerosol-forming material at low concentrations in order to significantly increase the local dielectric loss. When exposed to microwave energy, the sensitizer creates uniform heating of the aerosol forming material and increase efficiency of heating.

Preferably, a temperature of the aerosol-forming sample is measured or/and approximated and a value of which may be fed to a temperature control loop unit configured for controlling power supplied for energizing a microwave generation and/or releasing process.

It is of particular advantage to have an operation frequency which within a range of about 1 MHz to about 15 GHz, preferably in an ISM band, in a range of about 2.4 GHz to about 2.5 GHz and/or with a center frequency of about 2.45 GHz.

An underline operation power in continuous mode may be in a range from about 0.1 mW to 50 Watt and preferably around about 2 W.

Additionally or alternatively, an operation power in a pulsed mode may be in a range from about 0.1 mW to about 50 W and preferably around about 3 W.

An operation power may be ramped up between a value of about 0.1 W and a value of about 30 W.

As a further additional or alternative embodiment, an operation frequency may be set to a constant value.

An operation frequency may also be varied with time and in particular swept between a minimum value and the maximum value, in particular between about 2.4 GHz and about 2.5 GHz.

According to preferred embodiments of the present invention, an aerosol-forming sample may be used that is formed as, comprise, may be contained inside and/or may be packed inside at least one of a fluid material, a solid material, a tablet, capsule, cartridge, shell vial, pellet and/or can be fitted according to the size of an underlying cavity and/or pharmaceutical excipients are added to the aerosol-forming sample.

Further alternatively or additionally, said aerosol-forming sample may be or may form a vessel and/or may have a housing, wherein the housing in particular may be transparent with respect to microwave radiation, may comprise or may be formed of plastic, synthetic material, poly-propylene, glass, quartz and/or has a comparable low dielectric loss factor ε" and/or a comparable low loss tangent tan(δ).

As a further alternative or additional embodiment of the present invention, underlying capsule, cartridge or the like may be formed of or may comprise glass or quartz, in particular comprising ITO (indium tin oxide) at its ends, as ITO is electrically conducting and may therefore supply the high-frequency or microwave signal to the interior of the capsule. Said capsule may further in such an arrangement comprise 2 through holes with a ceiling in order to allow a gas stream or airstream to flow therethrough for releasing the aerosol generated from the inside.

Said aerosol-forming sample may be contained in a vessel formed as a mouthpiece which is configured for allowing inhalation of the formed aerosol, as an integral piece, as a disposal and/or to comprise a filter, in particular with the low or very low particulate filtration efficiency, and/or a hollow tube.

According to preferred embodiments of the present invention a plant-based material may be used which may be selected from the group comprising Cannabis sativa, Cannabis indica, Cannabis ruderalis, Acacia spp, Amanita muscaria, Yage, Atropa belladonna, Areca catechu, Brugmansia spp., Brunfelsia latifolia, Desmanthus illinoensis, Banisteriopsis caapi, Trichocereus spp., Theobroma cacao, Capsicum spp., Cestrum spp., Erythroxylum coca, Solenostemon scutellarioides, Arundo donax, Coffea arabica, Datura spp., Desfontainia spp., Diplopterys cabrerana, Ephedra sinica, Claviceps purpurea, Paullinia cupana, Argyreia nervosa, Hyoscyamus niger, Tabernanthe iboga, Lagochilus inebriens, Justicia pectoralis, Sceletium tortuosum, Piper methysticum, Catha edulis, Mitragyna speciosa, Leonotis leonurus, Nymphaea spp., Nelumbo spp., Sophora secundiflora, Mucuna pruriens, Mandragora officinarum, Mimosa tenuiflora, Ipomoea violacea, Psilocybe spp., Panaeolus spp., Myristica fragrans, Turbina corymbosa, Passiflora incarnata, Lophophora williamsii, Phalaris spp., Duboisia hopwoodii, Papaver somniferum, Psychotria viridis, spp., Salvia divinorum, Combretum quadrangulare, Trichocereus pachanoi, Heimia salicifolia, Stipa robusta, Solandra spp., Hypericum perforatum, Peganum harmala, Tabernaemontanaspp, Camellia sinensis, Nicotiana tabacum, rusticum, Virola theidora, Voacanga africana, Lactuca virosa, Artemisia absinthium, Ilex paraguariensis, Anadenanthera spp., Corynanthe yohimbe, Calea zacatechichi, Coffea spp. (Rubiaceae), a Sapindaceae, Camellia spp., Malvaceae spp., Aquifoliaceae spp., Hoodia, spp. Chamomilla recutita, Passiflora incarnate, Camellia sinensis, Mentha piperita, Mentha spicata, Rubus idaeus, Eucalyptus globulus, Lavandula officinalis, Thymus vulgaris, Melissa officinalis, Aloe Vera, Angelica, Anise, Ayahuasca (Banisteriopsis caapi), Barberry, Black Horehound, Blue Lotus, Burdock, Camomille/Chamomile, Caraway, Cat's Claw, Clove, Comfrey, Corn Silk, Couch Grass, Damiana, Damiana, Dandelion, Ephedra, Eucalyptus, Evening Primrose, Fennel, Feverfew, Fringe Tree, Garlic, Ginger, Ginkgo, Ginseng, Goldenrod, Goldenseal, Gotu Kola, Green Tea, Guarana, Hawthorn, Hops, Horsetail, Hyssop, Kola Nut, Kratom, Lavender, Lemon Balm, Licorice, Lion's Tail (Wild Dagga), Maca Root, Marshmallow, Meadowsweet, Milk Thistle, Motherwort, Passion Flower, Passionflower, Peppermint, Prickly Poppy, Purslane, Raspberry Leaf, Red Poppy, Sage, Saw Palmetto, Sida Cordifolia, Sinicuichi (Mayan Sun Opener), Spearmint, Sweet Flag, Syrian Rue (Peganum harmala), Thyme, Turmeric, Valerian, Wild Yam, Wormwood, Yarrow, Yerba Mate, Yohimbe, Cannabis sativa, Cannabis indica, and Cannabis ruderalis, any part and/or any combination thereof.

Additionally or alternatively a sample may be used which comprises and/or is capable of releasing a pharmacologically active agent of the sample and/or of the aerosol released is selected from the group comprising A9-tetrahydrocannabinol (THC), cannabidiol (CBD), cannabigerols (CBG), cannabichromenes (CBC), cannabinol (CBN), cannabinodiol (CBDL), cannabicyclol (CBL), cannabielsoin (CBE), cannabidivarin (CBDV), tetrahydrocannabivarin (THCV), cannabitriol (CBT) and combinations and parts thereof.

According to further additional or alternative preferred embodiments of the present invention within the inventions microwave heating method a sample may be used which comprises and/or is capable of releasing one or plural flavorants and/or sensates, said one or plural flavorants and/or sensates configured to generate taste and/or aroma, including any natural or synthetic flavorant, such as tobacco, smoke, menthol, mint, such as peppermint and spearmint, chocolate, licorice, citrus and other fruit flavorants, gamma octalactone, vanillin, ethyl vanillin, breath freshener flavorants, spice flavorants such as cinnamon, methyl salicylate, linalool, bergamot oil, geranium oil, lemon oil, and ginger oil, flavorant compounds selected from the group comprising of an acid, an alcohol, an ester, an aldehyde, a ketone, a pyrazine, combinations thereof and equivalents, from the group consisting of phenylacetic acid, solanone, megastigmatrienone, 2-heptanone, benzylalcohol, cis-3-hexenyl acetate, valeric acid, valeric aldehyde, ester, terpene, sesquiterpene, nootkatone, maltol, damascenone, pyrazine, lactone, anethole, iso-valeric acid, combinations thereof and equivalents, in encapsulated form for controlled delivery, peppermint, spearmint, wintergreen, menthol, cinnamon, chocolate, vanillin, licorice, clove, anise, sandalwood, geranium, rose oil, vanilla, lemon oil, cassia, spearmint, fennel, ginger, ethylacetate, isoamylacetate, propylisobutyrate, isobutylbutyrate, ethylbutyrate, ethylvalerate, benzylformate, limonene, cymene, pinene, linalool, geraniol, citronellol, citral, peppermint oil, orange oil, coriander oil, borneol, fruit extract, and equivalents. In a preferred embodiment, essential oils and essences of coffee, tea, cacao, and mint, a suitable amount of a flavorant present in core ranges from about 0.001 wt % to about 50 wt %, from about 1 wt % to about 40 wt %, from about 10 wt % to about 30 wt %, flavorant incorporated as a solid powder, sprayed dried as a liquid, or mixed with starch or gum-type matrix, and/or wherein any sensate is formed as or comprises one or plural an ingredients configured to order to induce a sensorial experience, such as tingling, sensation of warmth, sensation of cooling, and equivalents, and is or comprises at least one of an acetic acid, adipic acid, citric acid, lactic acid, maleic acid, succinic acid, tartaric acid, equivalents and mixtures thereof, with a suitable amount of a sensate agent in ranges of about 0.001 wt % to about 5 wt %, preferably of about 0.1 wt % to about 2 wt %, and/or their arbitrary combinations.

A sample may be used which comprises of tobacco, tobacco extracts and tobacco capsules, any raw or processed form of tobacco, as a powder, as a dust, a granule, a shred, a slurry, a flowable gel and equivalents, in particular with a final tobacco concentration ranging from 1 wt. % to 99 wt. % of a final composition and/or at most from about 10%, 15%, 20%, 25%, 30%, 35%, 40%, 45%, 50%, 55%, 60%, 65%, 70%, 75%, 80%, 85%, or 90% tobacco and/or one of the arbitrary combinations.

According to other preferred embodiments of the invention's microwave heating method sample may be used which comprises one or plural of humectants for maintaining and/or protecting moisture levels of the sample material and in particular of tobacco material in tobacco-containing hydrogel capsules and/or as preservatives to remove excess water and thereby, reduce the growth of micro-organisms, to provide a higher moisture feel in a drier sample material, tobacco material, tobacco substitute material and/or a drier smokeless tobacco material, comprising one or plural of glycerol and propylene glycol and/or in ranges from about 0.001 wt. % to about 5 wt. %, from about 0.1 wt. % to about 2 wt. %, and/or their arbitrary combinations.

According to other additional or alternative preferred embodiments of the invention's microwave heating method , a sample may be used which comprises a liquid, selected from the group of polyhydric alcohols consisting of glycerin, propylene glycol, and combinations thereof.

In the following, further alternative or additional preferred embodiments of the present invention in view of the involved aerosol-forming sample and the underlying material of the sample are presented:
An ingredient of the aerosol-forming sample and/or of the underlying material of the sample may comprise a drug of the composition of one of the following classes: antibiotics, anticonvulsants, antidepressants, antiemetics, antihistamines, antiparkinsonian drugs, antipsychotics, anxiolytics, drugs for erectile dysfunction, drugs for migraine headaches, drugs for the treatment of alcoholism, drugs for the treatment of addiction, muscle relaxants, nonsteroidal anti-inflammatories, opioids, other analgesics and stimulants.

Where the drug is an antibiotic, may be selected from one of the following compounds: cefmetazole; cefazolin; cephalexin; cefoxitin; cephacetrile; cephaloglycin; cephaloridine; cephalosporins, such as cephalosporin C; cephalotin; cephamycins, such as cephamycin A, cephamycin B, and cephamycin C; cepharin; cephradine; ampicillin; amoxicillin; hetacillin; carfecillin; carindacillin; carbenicillin; amylpenicillin; azidocillin; benzylpenicillin; clometocillin; cloxacillin; cyclacillin; methicillin; nafcillin; 2pentenylpenicillin; penicillins, such as penicillin N, penicillin 0, penicillin S, penicillin V; chiorobutin penicillin; dicloxacillin; diphenicillin; heptylpenidillin; and metampicillin.

Where the drug is an anticonvulsant, it may be selected from one of the following compounds: gabapentin, tiagabine, and vigabatrin.

Where the drug is an antidepressant, it may be selected from one of the following compounds: amitriptyline, amnoxapine, benmoxine, butriptyline, clomipramine, desipramine, dosulepin, doxepin, imipramine, kitanserin, lofepramine, medifoxamine, mianserin, maprotoline, mirtazapine, nortriptyline, protriptyline, trimipramine, viloxazine, citalopram, cotinine, duloxetine, fluoxetine, fluvoxamine, milnacipran, nisoxetine, paioxetine, reboxetine, sertraline, tianeptine, acetaphenazine, binedaline, brofaromine, cericlamine, clovoxamine, iproniazid, isocarboxazid, moclobemide, phenyhydrazine, pheneizine, selegiline, sibutramine, tranylcypromine, ademetionine, adrafinil, amesergide, amnisuipride, amperozide, benactyzine, bupropion, caroxazone, gepirone, idazoxan, metralindole, milnacipran, minaprine, nefazodone, noinifensine, ritanserin, roxindole, Sadenosylmethionine, tofenacin, trazodone, tryptophan, venlafaxine, and zalospirone.

Where the drug is an antiemetic, it may be selected from one of the following compounds: alizapride, azasetron, benzquinamide, bromopride, buclizine, chiorpromazine, cinnarizine, clebopride, cyclizine, diphenhydramine, cliphenidol, dolasetron methanesulfonate, droperidol, granisetron, hyoscine, lorazeparn, metoclopramide, metopimazine, ondansetron, perphenazine, promethazine, prochiorperazine, scopolamine, triethylperazine, trifluoperazine, triflupromazine, trimethobenzamide, tropisetron, domeridone, and palonosetron.

Where the drug is an antihistamine, it may be selected from one of the following compounds: azatadine, brompheniramine, chiorpheniramine, clemastine, cyproheptadine, dexmedetomidine, diphenhydramine, doxylamine, hydroxyzine, cetrizine, fexofenadine, loratidine, and promethazine.

Where the drug is an antiparkinsonian drug, it may be selected one of the following compounds: amantadine, baclofen, biperiden, benztropine, orphenadrine, procyclidine, trihexyphenidyl, levodopa, carbidopa, selegiline, deprenyl, andropinirole, apomnorphine, benserazide, bromocriptine, budipine, cabergoline, dihydroergokryptine, eliprodil, eptastigmine, ergoline pramipexole, galanthamine, lazabemide, lisuride, mazindol, memantine, mofegiline, pergolike, pramipexole, propentofylline, rasagiline, remacemide, spheramine, terguride, entacapone, and tolcapone.

Where the drug is an antipsychotic, it may be selected from one of the following compounds: acetophenazine, alizapride, anperozide, benperidol, benzquinamide, bromperidol, buramate, butaperazine, carphenazine, carpipramine, chlorpromazine, chiorprothixene, clocapramine, clomacran, clopenthixol, clospirazine, clothiapine, cyanemazine, droperidol, flupenthixol, fluphenazine, fluspirilene, haloperidol, mesoridazine, metofenazate, molindrone, penfluridol, pericyazine, perphenazine, pimozide, pipamerone, piperacetazine, pipotiazine, prochiorperazine, promazine, remoxipride, sertindole, spiperone, sulpiride, thioridazine, thiothixene, trifluperidol, triflupronazine, trifluoperazine, ziprasidone, zotepine, zuclopenthixol, anisuipride, butaclamol, clozapine, melperone, olanzapine, quetiapine, and risperidone.

Where the drug is an anxiolytic, it may be selected from one of the following compounds: mecloqualone, medetomidine, metomidate, adinazolai, chiordiazepoxide, clobenzepam, flurazepam, lorazepam, loprazulam, midazolam, alpidem, alseroxion, amphenidone, azacyclonol, bromisovalum, buspirone, calcium N-carboamoylaspartate, captodiamine, capuride, carbcloral, carbromal, chloral betaine, enciprazine, flesinoxan, ipsapiraone, lesopitron, loxapine, methaqualone, methprylon, propanolol, tandospirone, trazadone, zopiclone, and zolpidem.

Where the drug is a drug for erectile dysfunction, it may be selected from one of the following compounds: Cialis (IC351), sildenafil, vardenafil, apomorphine, apomorphine diacetate, phentolamine, and yohimbine.

Where the drug is a drug for migraine headache, it may be selected from one of the following compounds: almotriptan, alperopride, codeine, dihydroergotamine, ergotamine, eletriptan, frovatriptan, isometheptene, lidocaine, lisuride, metoclopramide, naratriptan, oxycodone, propoxyphene, rizatriptan, sumatriptan, tolfenamic acid, zolmitriptan, amitriptyline, atenolol, clonidine, cyproheptadine, diltiazem, doxepin, fluoxetine, lisinopril, methysergide, metoprolol, nadolol, nortriptyline, paroxetine, pizotifen, pizotyline, propanolol, protriptyline, sertraline, timolol, and verapamil.

Where the drug is a drug for the treatment of alcoholism, it may be selected from one of the following compounds: naloxone, naltrexone, and disulfiram.

Where the drug is a drug suitable for the treatment of addiction, it may be buprenorphine.

Where the drug is a muscle relaxant, it may be selected from one of the following compounds: baclofen, cyclobenzaprine, orphenadrine, quinine, and tizanidine.

Where the drug is a nonsteroidal anti-inflammatory, it may be selected from one of the following compounds: aceclofenac, alminoprofen, amfenac, aminopropylon, amixetrine, benoxaprofen, bromfenac, bufexamac, carprofen, choline, salicylate, cinchophen, cinmetacin, clopriac, clometacin, diclofenac, etodolac, indoprofen, mazipredone, meclofenamate, piroxicam, pirprofen, and tolfenamate.

Where the drug is an opioid, it may be selected from one of the following compounds: alfentanil, allylprodine, alphaprodine, anileridine, benzylmorphine, bezitramide, buprenorphine, butorphanol, carbiphene, cipramadol, clonitazene, codeine, dextromoramide, dextropropoxyphene, diamorphine, dihydrocodeine, diphenoxylate, dipipanone, fentanyl, hydromorphone, L-alpha acetyl methadol, lofentanil, levorphanol, meperidine, methadone, meptazinol, metopon, morphine, nalbuphine, nalorphine, oxycodone, papaveretum, pethidine, pentazocine, phenazocine, remifentanil, sufentanil, and tramadol.

Where the drug is another analgesic, it may be selected from one of the following compounds: apazone, benzpiperylon, benzydramine, caffeine, clonixin, ethoheptazine, flupirtine, nefopam, orphenadrine, propacetamol, and propoxyphene.

Where the drug is a stimulant, it may be selected from one of the following compounds: amphetamine, brucine, caffeine, dexfenfluramine, dextroamphetamine, ephedrine, fenfluramine, mazindol, methylphenidate, pemoline, phentermine, and sibutramine.

The drug may be selected from one of the following compounds: indoles, trypamines, benzofuranes, ibogoids, ergolines, phenetylamines, substituted phenethylamines, indane derivatives, benzocyclobuten derivatives, nBOMe derivatives, NBOH derivatives, NBMD derivatives, NBF derivatives, substituted amphetamines (alpha-methyl-phenethylamines): Substituted amphetamines (alpha-methyl-phenethylamines), DOx family (2,5-dimethoxy, 4-substituted amphetamines), phenylcyclopropylamine derivatives (technically not amphetamines), substituted methylenedioxy-phenethylamines (MDxx), substituted amphetamines, cathinones, substituted cathinones, benzofuranes, substituted benzofurans, tetraline, substituted tetralins, substituted indanes, substituted napthalenes, substituted phenylisobutylamines (alpha-ethyl-phenethylamines), alpha-substituted (-alkylated) tryptamines, arylcyclohexylamines, andamantanes, diarylethylamines, morphinans, opioids, benzodiazepines, thienodiazepines, GHB, GHB analogues, methaqualone, methaqualone analogues, synthetic cannabinoids, harmaline, salvinorines, Salvinorin A, Salvinorin B, Salvinorin C, Salvinorin D, Salvinorin E, Salvinorin F, Salvinorin G, Salvinorin H, Salvinorin I, 17a-Salvinorin J, 17β-Salvinorin J, piperazines, atropine derivatives, ibotenic acid, muscimol, psilocybin, ketamin, ketamin derivatives, oxytocin, nootropics, racteams, cocaine and cocaine analogues.

These aspects may be combined in an arbitrary manner.

These and further details, advantages and features of the present invention will be described based on embodiments of the invention and by taking reference to the accompanying figures.
- Figures 1 to 3: elucidate by means of schematic block diagrams preferred embodiments of the invention's microwave heating unit.
- Figures 4 to 10C: demonstrate details of preferred embodiments of the invention's microwave heating unit and its details.
- Figures 11 to 14: explain by means of cross-sectional side views other embodiments of the invention's microwave heating unit and its details.

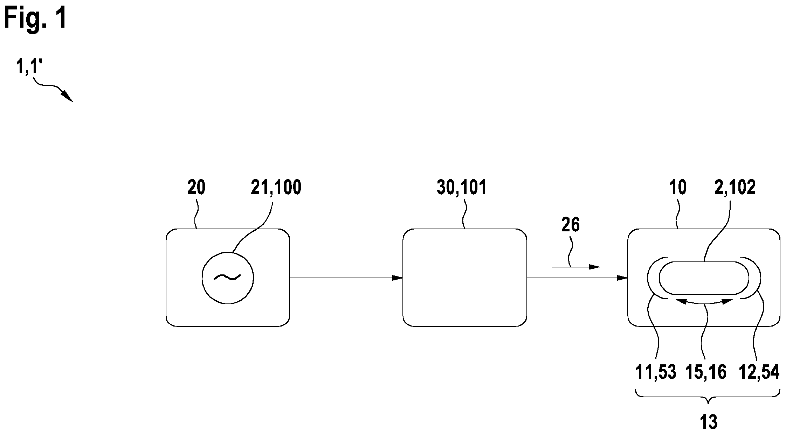

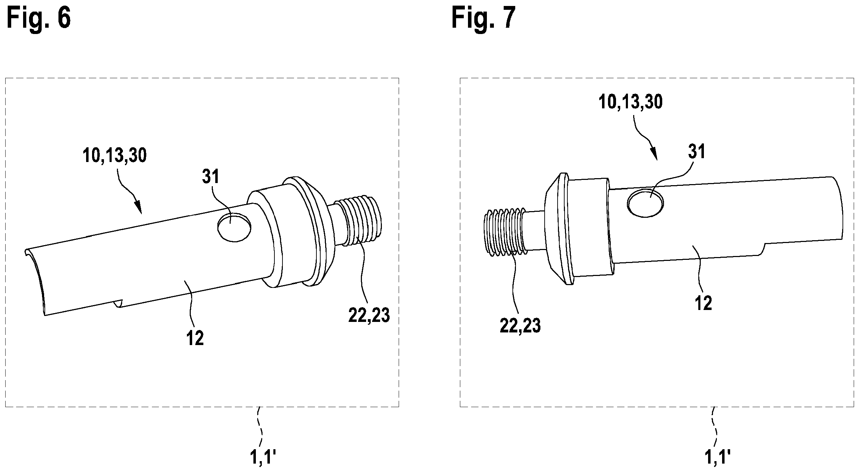

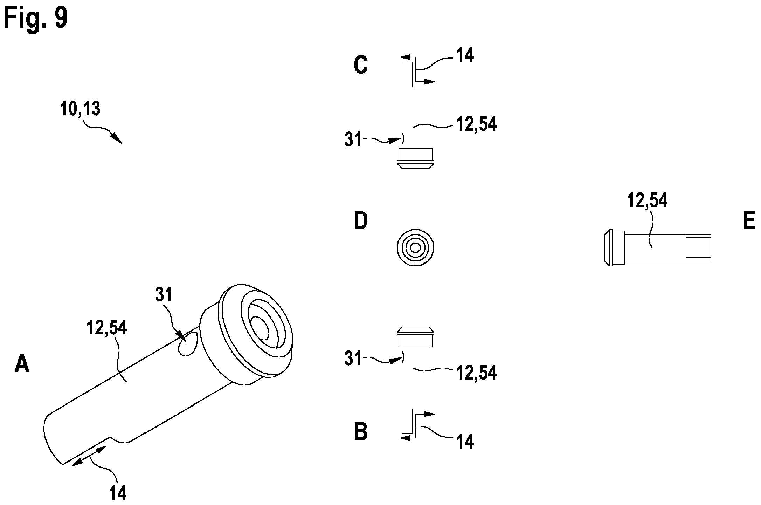

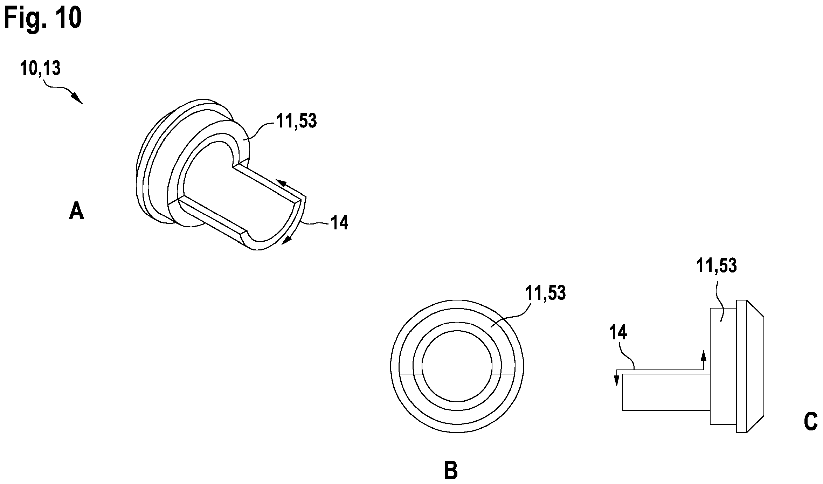

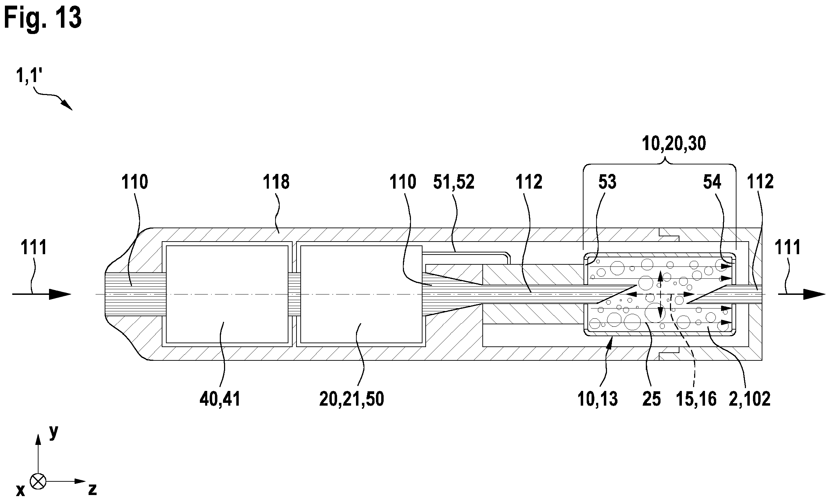

In the following embodiments and the technical background of the present invention are presented in detail by taking reference to accompanying figures1 to 14. Identical or equivalent elements and elements which act identically or equivalently are denoted with the same reference signs. Not in each case of their occurrence a detailed description of the elements and components is repeated.

The depicted and described features and further properties of the invention's embodiments can arbitrarily be isolated and recombined without leaving the gist of the present invention.

Figures 1 to 3 elucidate by means of schematic block diagrams preferred embodiments of the invention's microwave heating unit 1. Each of the microwave heating units or devices 1 shown in any of figures 1 to 3 may be formed as an inhalation, vaporizer or smoking product and/or device 1', in particular for medical and/or pulmonary drug delivery/inhalation functionalities.

The embodiment of the invention's microwave heating device or unit 1 as shown in figure 1 is formed by or comprises a microwave radiation generating and/or releasing unit 20, which might also be referred to as a microwave radiation signal generating and/or releasing unit in the sense of the present invention. Preferably, a solid-state microwave source 100 may be used in this regard as a microwave radiation source 20.

The thereby generated and/or provided microwave radiation field can be provided to a plant/pharmaceutical containing compound/material 102 as a sample to be contained in a holding and exposing space 15, for instance a cavity 16, formed by a sample holding and exposing means 10, for instance based on first and second holding parts or holding portions 11, 12.

This is preferably done by or in cooperation with an impedance matching unit 30 configured for matching the impedance between the provided microwave radiation field and the sample holder 13 defining the holding and exposing space 15 and its cavity 16.

In this regard, impedance matching may be achieved by at least one electrical or electronic means, geometrical means and/or material means. Electrical or electronic means refer to properties of the radiation and its underlying generating and/or supplying process. Geometrical means refer to positioning, geometry and/or orientation of certain physical items. Material means refer to the positioning, geometry and/or orientation of certain material items, for instance by using a dielectric material.

In this regard an impedance matching network 101 may be involved thereby realizing the impedance matching unit 30, the network 101 realizing one of the electric/electronic geometric and/or material aspects.

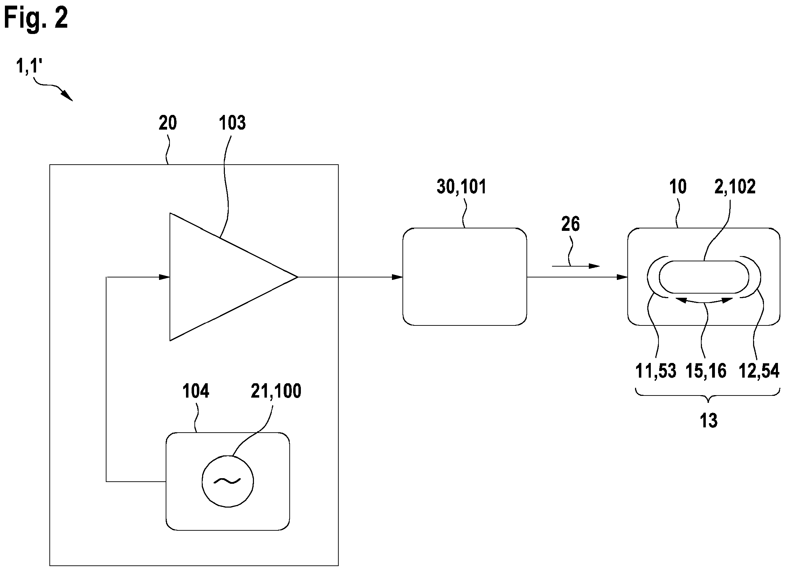

In the embodiment shown in figure 2 and in addition to the embodiment shown in figure 1, the microwave radiation generating and/or the releasing unit 20 may be formed by a consecutive arrangement of a small or low power signal source 104 comprising the microwave radiation source or microwave radiation signal source 21, for instance again as a solid-state microwave (signal) source 100, and a transistor amplifier 103.

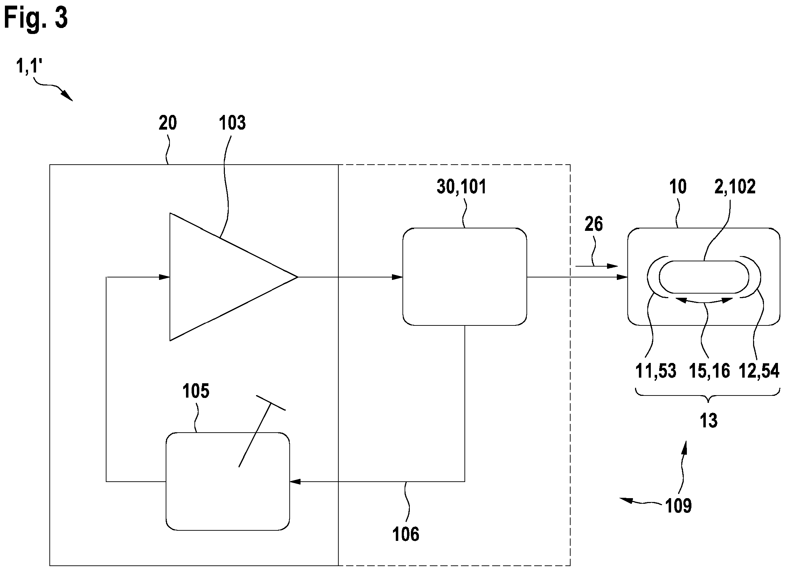

In the embodiment shown in figure 3 and in addition to the embodiment shown in figure 2, the microwave radiation generating and/or the releasing unit 20 comprises a feedback loop 106 connected between the impedance matching unit 30 and its network 101 giving input to an amplitude/phase correction device 105, with the output of which being fed into the input port of the transistor amplifier 103. Therefore, in the embodiment shown in figure 3 the small/low power signal source 104 substituted by the feedback loop 106 and the amplitude/phase correction device 105.

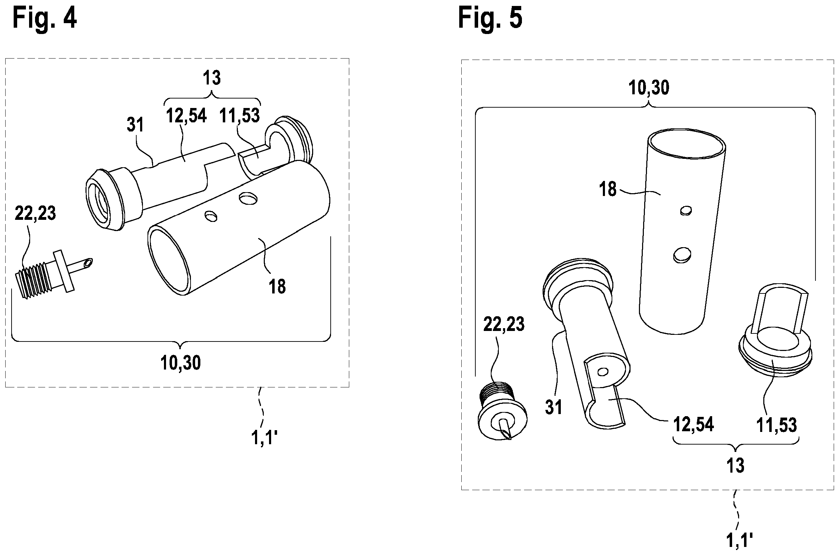

Figures 4 to 10C demonstrate details of preferred embodiments of the invention's microwave heating unit 1 and its details, each of which formed as an inhalation, vaporizer and/or smoking product and/or device 1', in particular for medical and/or pulmonary drug delivery/inhalation functionalities.

The invention's sample holding and exposing means 10 and the impedance matching unit 30 according to these embodiments are formed by first and second holding parts or holding portions 11 and 12 thereby defining a sample holder 13 for defining the holding and exposing space 15 by means of a cavity 16.

Essential aspects of the impedance matching unit 30 and its impedance matching network may be formed based on geometric and material aspects of the slits or slit structure 14 and in dielectric 17 used in this regard.

The sample holding and exposing means 10 may be surrounded by a housing 18 also yielding as a shield.

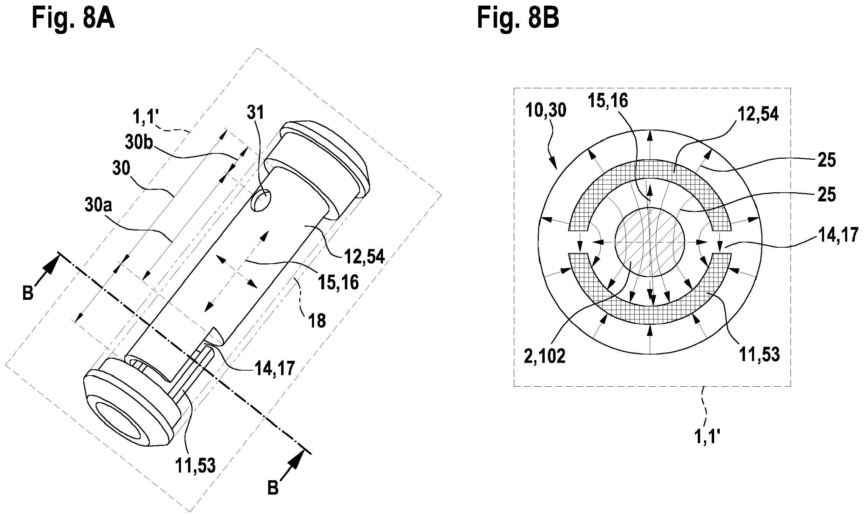

Figure 8B elucidates - by means of a cross-sectional view along a plane B-B indicated in figure 8A - details of the E-field configuration of the underlying microwave radiation field 25, namely in connection with the sample holding and exposing means 10, the impedance matching unit 30 and its arrangement of the slit structure 14 and the dielectric material 17 and also in connection with the first and second holding parts/portions which simultaneously serve as first and second electrodes 53 and 54, respectively.

As shown in figure 8A, the impedance matching unit 30 in one preferred embodiment may comprise a feeding portion of feeding point 31 by means of which microwave radiation may be introduced into the holding and exposing space 15 and the cavity 16. In this regard the first portion 30a and the second portion 30B of the sleeve component of the impedance matching unit 30 may have a length or dimension of about 1/8 λ to about 1/5 λ and less than 1/10 λ, wherein λ denotes the wavelength of the underlying microwave radiation.

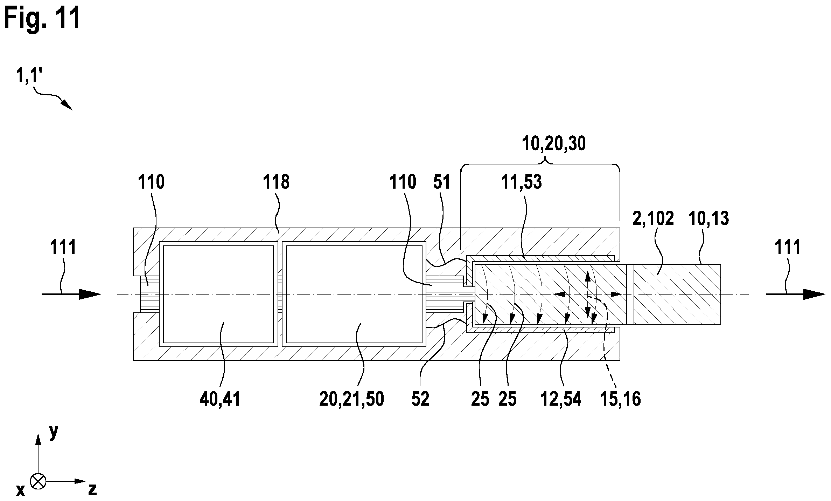

Figures 11 to 14 explain by means of cross-sectional side views other embodiments of the invention's microwave heating unit 1 and its details, each of which formed as an inhalation, vaporizer and/or smoking product and/or device 1', in particular for medical and/or pulmonary drug delivery/inhalation functionalities.

In each case of these embodiments, a power source unit 40 may be used which comprises a DC power source 40, for instance a battery, which is capable of energizing the underlying microwave radiation generating and/or releasing unit 20 with its microwave radiation source 21 and its control unit or control electronics 50 based on which and by means of first and second power supply lines 51, 52 first and second electrodes 53 and 54 are subjected to respective electric fields for generating the required microwave radiation field 25 within the cavity 16 of the holding and exposing space 15 formed in the sample holding and exposing means 10 of the sample holder 13.

By means of the microwave radiation field 25 the sample 2 with its sample material 102 - in the sense of a plant/pharmaceutical containing compound or material - is heated in a fast, reliable and uniform manner to thereby release an aerosol to be conveyed with an air flow 111 entering an air flow channel 110 formed in the main body or housing 118 of the device 1'.

The sample 2 and the sample material 102 may be designed in different forms.

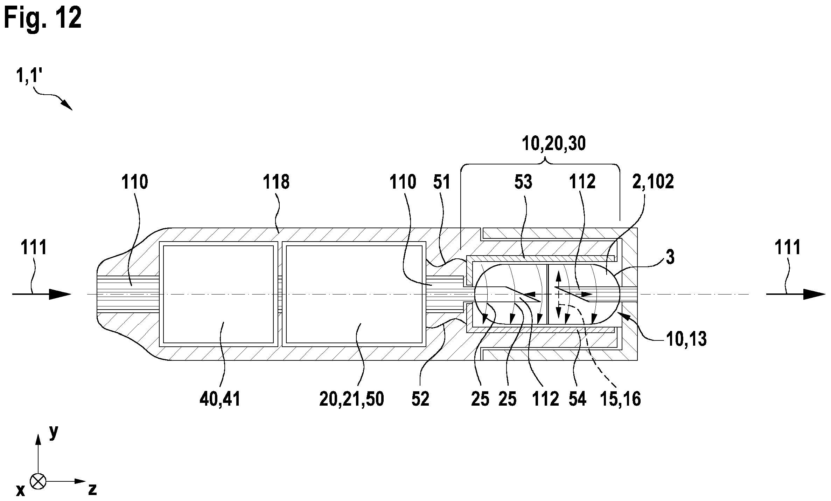

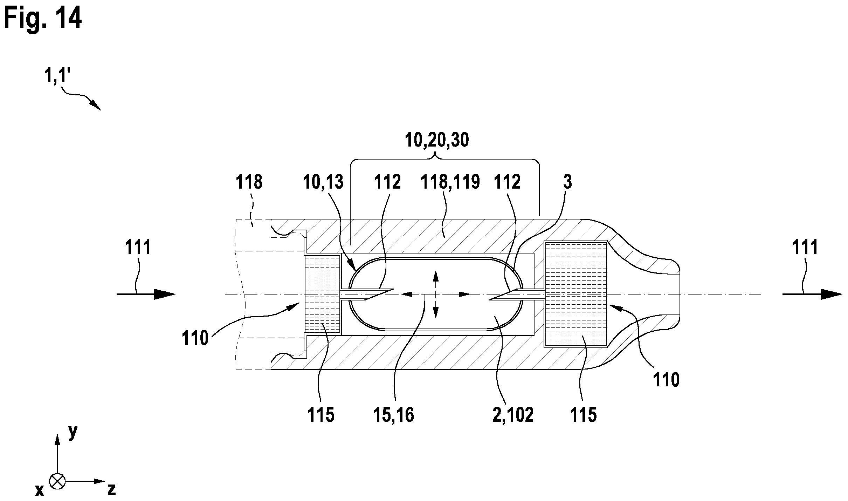

For instance, in the embodiments shown in figures 12 and 14 a capsule 3 is used formed by a more or less stable wall which - upon usage - has to be penetrated by means of needles 112 or any other penetration means contained and formed in the air flow channel 110 of the device 1'.

As shown in figure 14, at least a part of the main body 118 or its housing 118 may be formed as a mouthpiece 119, optionally equipped with filter components 115 for interacting with the air flow 111 in the air flow channel 110.

### List of reference signs

- 1: microwave heating device/unit
- 1': inhalation, vaporizer and/or smoking product and/or device
- 2: sample
- 10: sample holding and exposing means
- 11: 1^{st} holding part/holding portion
- 12: 2^{nd} holding part/holding portion
- 13: sample holder
- 14: slit, slit structure
- 15: holding and exposing space
- 16: cavity
- 17: dielectric material portion
- 18: housing, shield
- 20: microwave radiation generating and/or releasing unit, microwave radiation signal generating and/or releasing unit
- 21: microwave radiation source, solid-state microwave source, microwave radiation signal source, solid-state microwave signal source
- 22: waveguide
- 23: connector
- 25: microwave radiation field
- 26: microwave radiation field signal
- 30: impedance matching unit
- 30a: first portion of impedance matching unit 30
- 30b: second portion of impedance matching unit 30
- 31: feeding point for microwave radiation field 25
- 40: power source unit
- 41: DC power source, battery
- 50: control unit, electronics
- 51: 1^{st} power supply line
- 52: 2^{nd} power supply line
- 53: 1^{st} electrode
- 54: 2^{nd} electrode

- 100: solid-state microwave source, solid-state microwave signal source
- 101: impedance matching network
- 102: sample material, plant/pharmaceutical containing compound/material
- 103: transistor amplifier
- 104: small/low power signal source
- 105: amplitude/phase correction device
- 106: feedback loop/branch/line
- 109: (free running) oscillator
- 110: air flow channel
- 111: air flow
- 112: needle, penetration means
- 115: Filter
- 118: main body, housing
- 119: mouthpiece

- x: spatial direction
- y: spatial direction
- z: spatial direction

## Claims

1. Microwave heating unit (1) for heating an aerosol-forming sample (2) due to microwave absorption by a material (102) of the sample (2) and for thereby releasing by or from said sample (2) upon heating said sample (2) at least one aerosol, for use in a or as an inhalation, vaporizer and/or smoking product or device (1') and/or for pulmonary drug delivery, wherein the microwave heating unit (1) comprises:
(i) a sample holding and exposing unit (10) configured to receive and hold a sample (2) in a holding and exposing space (15) and to expose the sample (2) to a microwave radiation field (25) within said holding and exposing space (15),
(ii) a microwave radiation unit (20) configured to supply said microwave radiation field (25) and/or an underlying microwave radiation signal (26) to said holding and exposing space (15), and
(iii) an impedance matching unit (30) which is configured to achieve impedance matching between the holding and exposing space (15) and the signal (26) and/or the microwave radiation field (25) wherein the impedance matching unit (30) and in particular an impedance matching network (101) thereof comprises electrodes (53, 54) for generating the microwave radiation field (25) upon receipt of the underlying microwave radiation signal (26),
wherein said sample holding and exposing unit (10) comprises a sample holder (13) defining and forming said holding and exposing space (15) within an interior, in particular as a cavity (16), by means of first and second holding portions (11, 12) and/or first and second holding portions separated by a slit structure (14),
and wherein the first and second holding portions (11, 12) simultaneously serve as first and second electrodes (53, 54), respectively.

2. Microwave heating unit (1) according to claim 1, comprising a free running oscillator (109) which is configured to generate and supply said microwave radiation field (25) and/or said underlying microwave radiation signal (26).

3. Microwave heating unit (1) according to any one of the preceding claims, wherein the impedance matching unit (30) and in particular an impedance matching network (101) thereof is tunable in its matching properties concerning at least one of impedance, frequency and the aerosol-forming sample (2).

4. Microwave heating unit (1) according to any one of the preceding claims, wherein the impedance matching unit (30) and in particular an impedance matching network (101) thereof comprises at least one of
- the electrodes (53, 54) for generating the microwave radiation field (25) upon receipt of the underlying microwave radiation signal (26), which geometrically fit to the aerosol-forming sample (2), holding and exposing space (15) and/or a or the cavity (16) thereof,
- an output which contains waveguide structures, transmission lines and/or stubs,
- 1/8 λ to 1/4 λ wave guide matching elements and
- one or plural dielectric material portions (17),
by means of which the impedance matching unit (30) is in particular tunable in its properties.

5. Microwave heating unit (1) according to any one of the preceding claims, wherein
- said microwave radiation unit (20) is or comprises one or plural microwave radiation or radiation signal sources (21) - in particular in the form of solid-state microwave or radiation signal sources - and/or a connecting configuration with a connector (23) and/or a waveguide (22) for providing a connection to one or plural microwave radiation sources (21) and
- said one or plural microwave radiation or radiation signal sources (21) and solid-state microwave or radiation signal sources are configured upon its energization to generate and/or release said underlying microwave radiation field (25) or the underlying signal (26).

6. Microwave heating unit (1) according to claim 5, comprising a power source unit (40) which is configured to energize the underlying microwave radiation unit (20) and in particular the one or plural microwave radiation or radiation signal sources (21) and which in particular comprises one or plural DC power sources (41).

7. Microwave heating unit (1) according to any one of the preceding claims, wherein
- said microwave radiation unit (20) and in particular a microwave radiation or radiation signal source (21) thereof is or comprises a transistor amplifier (103) and/or
- said impedance matching unit (30) and in particular an impedance matching network (101) thereof comprises a microwave feedback port and/or a microwave signal feedback port which is connected to an input of the transistor amplifier (103) in order to meet an underlying oscillation condition.

8. Microwave heating unit (1) according to claim 7, wherein an additional smart phase and/or an amplitude correction device (105) is or are connected in between the feedback port and the input of the transistor amplifier (103).

9. Microwave heating unit (1) according to any one of the preceding claims, wherein
- the microwave radiation unit (20) and in particular a microwave radiation source (21) and/or a microwave radiation signal source (21) thereof is or comprises a transistor amplifier (103) and in particular
- has an input that is connected to an output of a low power signal source (104) and/or
- the low power signal source (104) is or comprises at least one of a phased locked loop (PLL), a voltage-controlled oscillator (VCO) and a direct digital synthesis (DDS).

10. Microwave heating unit (1) according to any one of the preceding claims, which is formed in or as at least one of a drug delivery product and/or device, an inhalation product or device (1'), smoking product or device (1'), a mobile product or device (1') and/or a portable product or device (1').

11. Microwave heating unit (1) according to any one of the preceding claims, wherein said free running oscillator (109) at least one of
- is connected to the sample holding and exposing unit (10) or is a part thereof,
- is, comprises and/or realizes an IQ modulator and/or the functionality of an IQ modulator which in particular means the ability of controlling the phase and/or the gain and
- comprises within a feedback loop (106) a or the transistor amplifier (103), an or the impedance matching unit (30) and/or impedance matching network (101) and a or the amplitude/phase correction device (105).

12. Microwave and/or RF heating method for heating an aerosol-forming sample (2) due to microwave absorption by a material (102) of said sample (2) and for thereby releasing by or from said sample (2) upon heating said sample (2) at least one aerosol, the at least one aerosol comprising at least one of a phyto-active substance and a pharmaceutical active ingredient,
- wherein a microwave heating unit (1) according to any one of the preceding claims is used for heating said sample (2) and
- wherein in particular at least one of
- an aerosol-forming substrate (102) of a smoking article, in particular of a nicotine and/or non-nicotine containing smoking article,
- a tobacco-loaded solid-aerosol forming sample of a smoking sample, in particular of a nicotine and/or non-nicotine containing smoking sample,
- an aerosol-forming substrate with or of a nicotine and/or non-nicotine containing liquid,
- an aerosol-forming active pharmaceutical ingredient,
- an aerosol-forming phyto-active substance and in particular from raw plant material and/or from a plant material,
- an aerosol-forming delta-9-Tetrahydrocanabinol (THC) and/or cannabidiol (CBD) and/or cannabinoids containing plant material,
- an aerosol-forming botanical drug substance and/or a botanical drug product,
- an aerosol-forming substrate (102) having at least one of microwave sensitizers and/or absorbers of the group of substances which comprises functionalized polysilsesquioxanes, carbon nanotubes, graphite, graphene, activated carbon, activated charcoal, metal powder, semiconductor powder and combinations and mixtures thereof,
is or are used and/or heated as said sample (2) or as a part thereof.

13. Microwave heating method according to claim 12, wherein a temperature of the aerosol-forming sample (2) is measured or/and approximated and a value of which is fed to a temperature control loop unit controlling power supplied for energizing a microwave generation and/or releasing process.

14. Microwave heating method according to any one of claims 12 and 13, wherein:
- an operation frequency is in the range from about 1 MHz to about 15 GHz, preferably in an ISM band and/or in a range of about 2.4 GHz to about 2.5 GHz and/or with a center frequency of about 2.45 GHz,
- an operation power in continuous mode is in a range from about 0.1 mW to 50 Watt and preferably around about 2 W,
- an operation power in a pulsed mode is in a range from about 0.1 mW to about 50 W and preferably around about 3 W,
- an operation power is ramped up between a value of about 0.1 W and a value of about 30 W, and/or
- an operation frequency is set to a constant value,
- an operation frequency is varied with time and in particular swept between a minimum value and the maximum value, in particular between about 2.4 GHz and about 2.5 GHz.

15. Microwave heating method according to any one of claims 12 to 14, wherein an aerosol-forming sample (2) is used which at least one of
- is formed as, comprises, is contained inside and/or is packed inside at least one of a fluid material, a solid material, a tablet, capsule, cartridge, shell vial, pellet and/or can be fitted according to the size of an underlying cavity (16) and/or pharmaceutical excipients are added to the aerosol-forming sample (2),
- is or forms a vessel and/or has a housing, wherein the housing in particular is transparent with respect to microwave radiation, comprises or is formed of plastic, synthetic material, poly-propylene, glass, quartz and/or has a comparable low dielectric loss factor (ε") and/or a comparable low loss tangent (tan(*δ))*, and
- is contained in a vessel formed as a mouthpiece (119) which is configured for allowing inhalation of the formed aerosol, as an integral piece, as a disposal and/or to comprise a filter (115), in particular with the low or very low particulate filtration efficiency, and/or a hollow tube.

## Patentansprüche

1. Mikrowellenheizeinheit (1) zum Erhitzen einer aerosolbildenden Probe (2) aufgrund einer Mikrowellenabsorption durch ein Material (102) der Probe (2) und zum dadurch Freisetzen mindestens eines Aerosols durch oder aus der Probe (2) beim Erhitzen der Probe (2), zur Verwendung in einem oder als Inhalations-, Verdampfer- und/oder Raucherprodukt oder -vorrichtung (1') und/oder zur pulmonalen Arzneimittelabgabe, wobei die Mikrowellenheizeinheit (1) umfasst:
(i) eine Probehalte- und Aussetzungseinheit (10), die konfiguriert ist, um eine Probe (2) in einen Halte- und Aussetzungsraum (15) aufzunehmen und zu halten und um die Probe (2) einem Mikrowellenstrahlungsfeld (25) innerhalb des Halte- und Aussetzungsraums (15) auszusetzen,
(ii) eine Mikrowellenstrahlungseinheit (20), die konfiguriert ist, um das Mikrowellenstrahlungsfeld (25) und/oder ein zugrundeliegendes Mikrowellenstrahlungssignal (26) an dem Halte- und Aussetzungsraum (15) bereitzustellen, und
(iii) eine Impedanzanpassungseinheit (30), die konfiguriert ist, um eine Impedanzanpassung zwischen dem Halte- und Aussetzungsraum (15) und dem Signal (26) und/oder dem Mikrowellenstrahlungsfeld (25) zu ermöglichen, wobei die Impedanzanpassungseinheit (30) und insbesondere ein Impedanzanpassungsnetzwerk (101) davon Elektroden (53, 54) zum Erzeugen des Mikrowellenstrahlungsfeldes (25) bei Empfang des zugrundeliegenden Mikrowellenstrahlungssignals (26) umfasst,
wobei die Probehalte- und Aussetzungseinheit (10) einen Probenhalter (13) umfasst, der den Halte- und Aussetzungsraum (15) innerhalb eines Innenraums, insbesondere als Hohlraum (16), mittels erster und zweiter Halteabschnitte (11, 12) und/oder erster und zweiter Halteabschnitte, die durch eine Schlitzstruktur (14) getrennt sind, definiert und bildet, und
wobei die ersten und zweiten Halteabschnitte (11, 12) simultan als erste und zweite Elektroden (53, 54) dienen.

2. Mikrowellenheizeinheit (1) nach Anspruch 1, umfassend einen freilaufenden Oszillator (109), der konfiguriert ist, um das Mikrowellenstrahlungsfeld (25) und/oder das zugrundeliegende Mikrowellenstrahlungssignal (26) zu erzeugen und bereitzustellen.

3. Mikrowellenheizeinheit (1) nach einem der vorhergehenden Ansprüche, wobei die Impedanzanpassungseinheit (30) und insbesondere ein Impedanzanpassungsnetzwerk (101) davon in seinen Anpassungseigenschaften bezüglich der Impedanz, der Frequenz und/oder der aerosolbildenden Probe (2) einstellbar ist.

4. Mikrowellenheizeinheit (1) nach einem der vorhergehenden Ansprüche, wobei die Impedanzanpassungseinheit (30) und insbesondere ein Impedanzanpassungsnetzwerk (101) davon mindestens umfasst:
- die Elektroden (53, 54) zur Erzeugung des Mikrowellenstrahlungsfeldes (25) bei Empfang des zugrundeliegenden Mikrowellenstrahlungssignals (26), die geometrisch an die aerosolbildende Probe (2), den Halte- und Aussetzungsraum (15) und/oder einen oder den Hohlraum (16) davon angepasst sind,
- und/oder einen Ausgang, der Wellenleiterstrukturen, Übertragungsleitungen und/oder Stummel enthält,
- und/oder 1/8 λ bis 1/4 λ Wellenleiteranpassungselemente,
- und/oder ein oder mehrere Abschnitte aus dielektrischem Material (17),
mittels derer die Impedanzanpassungseinheit (30) insbesondere in ihren Eigenschaften einstellbar ist.

5. Mikrowellenheizeinheit (1) nach einem der vorhergehenden Ansprüche, wobei
- die Mikrowellenstrahlungseinheit (20) eine oder mehrere Mikrowellenstrahlungs- oder Strahlungssignalquellen (21) - insbesondere in Form von Festkörpermikrowellen- oder Strahlungssignalquellen - und/oder eine Verbindungskonfiguration mit einem Verbinder (23) und/oder einem Wellenleiter (22) zur Bereitstellung einer Verbindung mit einer oder mehreren Mikrowellenstrahlungsquellen (21) ist oder umfasst, und
- die eine oder mehrere Mikrowellenstrahlungs- oder Strahlungssignalquellen (21) und Festkörpermikrowellen- oder Strahlungssignalquellen bei ihrer Bestromung konfiguriert sind, um das zugrundeliegende Mikrowellenstrahlungsfeld (25) oder das zugrundeliegende Signal (26) zu erzeugen und/oder freizusetzen.

6. Mikrowellenheizeinheit (1) nach Anspruch 5, umfassend eine Stromquelleneinheit (40), die zur Bestromung der zugrundeliegenden Mikrowellenstrahlungseinheit (20) und insbesondere der einen oder mehreren Mikrowellenstrahlungs- oder Strahlungssignalquellen (21) konfiguriert ist und insbesondere eine oder mehrere Gleichstromquellen (41) umfasst.

7. Mikrowellenheizeinheit (1) nach einem der vorhergehenden Ansprüche, wobei
- die Mikrowellenstrahlungseinheit (20) und insbesondere eine Mikrowellenstrahlungs- oder Strahlungssignalquelle (21) davon ein Transistorverstärker (103) ist oder umfasst, und/oder
- die Impedanzanpassungseinheit (30) und insbesondere ein Impedanzanpassungsnetzwerk (101) davon einen Mikrowellenrückkopplungsanschluss und/oder einen Mikrowellensignalrückkopplungsanschluss umfasst, der mit einem Eingang des Transistorverstärkers (103) verbunden ist, um eine zugrundeliegende Oszillationsbedingung zu erfüllen.

8. Mikrowellenheizeinheit (1) nach Anspruch 7, wobei eine zusätzliche intelligente Phasen- und/oder Amplitudenkorrekturvorrichtung (105) zwischen dem Rückkopplungsanschluss und dem Eingang des Transistorverstärkers (103) angeschlossen ist oder sind.

9. Mikrowellenheizeinheit (1) nach einem der vorhergehenden Ansprüche,
- wobei die Mikrowellenstrahlungseinheit (20) und insbesondere eine Mikrowellenstrahlungsquelle (21) und/oder eine Mikrowellenstrahlungssignalquelle (21) davon ein Transistorverstärker (103) ist oder umfasst, und insbesondere
- einen Eingang umfasst, der mit einem Ausgang einer Niederleistungssignalquelle (104) verbunden ist, und/oder
- wobei die Niederleistungssignalquelle (104) eine Phasenregelschleife (PLL), ein spannungsgesteuerter Oszillator (VCO) und/oder eine direkte digitale Synthese (DDS) ist oder umfasst.

10. Mikrowellenheizeinheit (1) nach einem der vorhergehenden Ansprüche, die in oder als Arzneimittelabgabeprodukt und/oder -vorrichtung, Inhalationsprodukt oder -vorrichtung (1'), Rauchprodukt oder -vorrichtung (1'), mobiles Produkt oder Vorrichtung (1') und/oder tragbares Produkt oder Vorrichtung (1') ausgeformt ist.

11. Mikrowellenheizeinheit (1) nach einem der vorhergehenden Ansprüche, wobei der freilaufende Oszillator (109)
- mit der Probehalte- und Aussetzungseinheit (10) verbunden ist oder ein Teil davon ist,
- und/oder ein IQ-Modulator und/oder die Funktionalität eines IQ-Modulators ist, umfasst und/oder ermöglicht, was insbesondere die Fähigkeit zur Steuerung der Phase und/oder der Verstärkung bedeutet,
- und/oder innerhalb eines Rückkopplungskreises (106) einen oder den Transistorverstärker (103), eine oder die Impedanzanpassungseinheit (30) und/oder ein Impedanzanpassungsnetzwerk (101) und eine oder die Amplituden-/Phasenkorrekturvorrichtung (105) umfasst.

12. Mikrowellen- und/oder RF-Heizverfahren zum Erhitzen einer aerosolbildenden Probe (2) aufgrund einer Mikrowellenabsorption durch ein Material (102) der Probe (2) und zum dadurch Freisetzen durch oder aus der Probe (2) beim Erhitzen der Probe (2) mindestens eines Aerosols, wobei das mindestens eine Aerosol mindestens eine phytoaktive Substanz und einen pharmazeutischen aktiven Inhaltsstoff umfasst,
- wobei zum Erwärmen der Probe (2) eine Mikrowellenheizeinheit (1) nach einem der vorhergehenden Ansprüche verwendet wird, und
- wobei insbesondere
- ein aerosolbildendes Substrat (102) eines Rauchartikels, insbesondere eines nikotin- und/oder nicht-nikotinhaltigen Rauchartikels,
- und/oder eine tabakbeladene, feste-aerosolbildende Probe einer Rauchprobe, insbesondere einer nikotin- und/oder nicht-nikotinhaltigen Rauchprobe,
- und/oder ein aerosolbildendes Substrat mit oder von einer nikotin- und/oder nicht-nikotinhaltigen Flüssigkeit,
- und/oder ein aerosolbildender aktiver pharmazeutischer Inhaltsstoff,
- und/oder eine aerosolbildende phytoaktive Substanz, insbesondere aus pflanzlichem Rohmaterial und/oder aus einem Pflanzenmaterial,
- und/oder ein aerosolbildendes delta-9-Tetrahydrocanabinol (THC) und/oder Cannabidiol (CBD) und/oder cannabinoidhaltiges Pflanzenmaterial,
- und/oder einen aerosolbildenden botanischen Arzneistoff und/oder ein botanisches Arzneimittel,
- und/oder ein aerosolbildendes Substrat (102) mit mindestens einem Mikrowellensensibilisator und/oder Absorber der Substanzgruppe, die funktionalisierte Polysilsesquioxane, Kohlenstoffnanoröhren, Graphit, Graphen, aktivierten Kohlenstoff, aktivierte Holzkohle, Metallpulver, Halbleiterpulver und Kombinationen und Mischungen davon umfasst,
als genannte Probe (2) oder als ein Teil davon verwendet und/oder erhitzt wird oder werden.

13. Mikrowellenheizverfahren nach Anspruch 12, wobei eine Temperatur der aerosolbildenden Probe (2) gemessen oder/und approximiert wird und ein Wert davon einer Temperaturregelkreiseinheit zugeführt wird, die den Strom steuert, der zur Bestromung eines Mikrowellenerzeugungs- und/oder -freisetzungsprozesses bereitgestellt wird.

14. Mikrowellenheizverfahren nach Anspruch 12 oder 13, wobei:
- eine Betriebsfrequenz im Bereich von etwa 1 MHz bis etwa 15 GHz liegt, vorzugsweise in einem ISM-Band und/oder in einem Bereich von etwa 2,4 GHz bis etwa 2,5 GHz und/oder mit einer Mittenfrequenz von etwa 2,45 GHz,
- eine Betriebsleistung im Dauermodus in einem Bereich von etwa 0,1 mW bis 50 Watt und vorzugsweise bei etwa 2 W liegt,
- eine Betriebsleistung in einem gepulsten Modus in einem Bereich von etwa 0,1 mW bis etwa 50 W und vorzugsweise etwa 3 W liegt,
- eine Betriebsleistung zwischen einem Wert von etwa 0,1 W und einem Wert von etwa 30 W erhöht wird,
- eine Betriebsfrequenz auf einen konstanten Wert eingestellt wird, und/oder
- eine Betriebsfrequenz mit der Zeit variiert und insbesondere zwischen einem Minimalwert und dem Maximalwert, insbesondere zwischen etwa 2,4 GHz und etwa 2,5 GHz, schwingt.

15. Mikrowellenheizverfahren nach einem der Ansprüche 12 bis 14, wobei eine aerosolbildende Probe (2) verwendet wird, die
- als ein flüssiges Material, ein festes Material, eine Tablette, eine Kapsel, eine Patrone, ein Shell-Vial und/oder ein Pellet ausgeformt ist, umfasst, darin aufgenommen ist und/oder darin verpackt ist und/oder entsprechend der Größe eines zugrundeliegenden Hohlraums (16) eingepasst werden kann und/oder der aerosolbildenden Probe (2) pharmazeutische Hilfsstoffe hinzugefügt werden,
- und/oder ein Behälter ist oder bildet und/oder ein Gehäuse aufweist, wobei das Gehäuse insbesondere für Mikrowellenstrahlung transparent ist, Kunststoff, synthetisches Material, Polypropylen, Glas, Quarz umfasst oder daraus ausgeformt ist, und/oder einen vergleichbar niedrigen dielektrischen Verlustfaktor (ε") und/oder einen vergleichbar niedrigen Verlusttangens (tan(*δ*)) umfasst,
- und/oder in einem als Mundstück (119) ausgeformten Behälter aufgenommen ist, der konfiguriert ist, um die Inhalation des gebildeten Aerosols zu ermöglichen, als ein integrales Stück, als ein Auswechselstück und/oder zum Aufnehmen eines Filters (115), insbesondere mit der niedrigen oder sehr niedrigen Partikelfiltereffizienz, und/oder als ein Hohlrohr.

## Revendications

1. Unité de chauffage par micro-ondes (1) pour chauffer un échantillon de formation d'aérosol (2) en raison de l'absorption des micro-ondes par un matériau (102) de l'échantillon (2) et pour libérer ainsi par ou à partir dudit échantillon (2) lors du chauffage dudit échantillon (2) au moins un aérosol, pour une utilisation dans un ou comme un produit ou dispositif d'inhalation, de vaporisation et/ou à fumer (1') et/ou pour une administration de médicament pulmonaire, dans laquelle l'unité de chauffage par micro-ondes (1) comprend :
(i) une unité de maintien et d'exposition d'échantillon (10) configurée pour recevoir et maintenir un échantillon (2) dans un espace de maintien et d'exposition (15) et pour exposer l'échantillon (2) à un champ de rayonnement micro-ondes (25) à l'intérieur dudit espace de maintien et d'exposition (15),
(ii) une unité de rayonnement micro-ondes (20) configurée pour fournir ledit champ de rayonnement micro-ondes (25) et/ou un signal de rayonnement micro-ondes sous-jacent (26) audit espace de maintien et d'exposition (15), et
(iii) une unité d'adaptation d'impédance (30) qui est configurée pour réaliser une adaptation d'impédance entre l'espace de maintien et d'exposition (15) et le signal (26) et/ou le champ de rayonnement micro-ondes (25) dans laquelle l'unité d'adaptation d'impédance (30) et en particulier un réseau d'adaptation d'impédance (101) de celle-ci comprend des électrodes (53, 54) pour générer le champ de rayonnement micro-ondes (25) lors de la réception du signal de rayonnement micro-ondes sous-jacent (26),
dans laquelle ladite unité de maintien et d'exposition d'échantillon (10) comprend un porte-échantillon (13) définissant et formant ledit espace de maintien et d'exposition (15) dans un intérieur, en particulier comme une cavité (16), au moyen de première et deuxième parties de maintien (11, 12) et/ou de première et deuxième parties de maintien séparées par une structure à fentes (14),
et dans laquelle les première et deuxième parties de maintien (11, 12) servent simultanément de première et deuxième électrodes (53, 54), respectivement.

2. Unité de chauffage par micro-ondes (1) selon la revendication 1, comprenant un oscillateur d'oscillations libres (109) qui est configuré pour générer et fournir ledit champ de rayonnement micro-ondes (25) et/ou ledit signal de rayonnement micro-ondes sous-jacent (26).

3. Unité de chauffage par micro-ondes (1) selon l'une quelconque des revendications précédentes, dans laquelle l'unité d'adaptation d'impédance (30) et en particulier un réseau d'adaptation d'impédance (101) de celle-ci est accordable dans ses propriétés d'adaptation concernant au moins l'un parmi l'impédance, la fréquence et l'échantillon de formation d'aérosol (2).

4. Unité de chauffage par micro-ondes (1) selon l'une quelconque des revendications précédentes, dans laquelle l'unité d'adaptation d'impédance (30) et en particulier un réseau d'adaptation d'impédance (101) de celle-ci comprend au moins l'un
- des électrodes (53, 54) pour générer le champ de rayonnement micro-ondes (25) lors de la réception du signal de rayonnement micro-ondes sous-jacent (26), qui s'adapte géométriquement à l'échantillon de formation d'aérosol (2), à l'espace de maintien et d'exposition (15) et/ou à une ou la cavité (16) de celui-ci,
- d'une sortie qui contient des structures de guide d'ondes, des lignes et/ou des tronçons de transmission,
- des éléments d'adaptation de guide d'ondes de 1/8 λ à 1/4 λ et
- d'une ou de plusieurs partie(s) de matériau diélectrique (17),
au moyen duquel l'unité d'adaptation d'impédance (30) est en particulier accordable dans ses propriétés.

5. Unité de chauffage par micro-ondes (1) selon l'une quelconque des revendications précédentes, dans laquelle
- ladite unité de rayonnement micro-ondes (20) est ou comprend une ou plusieurs source(s) de signal de rayonnement ou de rayonnement micro-ondes (21) - en particulier sous forme de sources de signal de rayonnement ou micro-ondes à semi-conducteurs - et/ou une configuration de connexion avec un connecteur (23) et/ou un guide d'ondes (22) pour fournir une connexion à une ou plusieurs source(s) de rayonnement micro-ondes (21) et
- ladite/lesdites une ou plusieurs source(s) de signal de rayonnement ou de rayonnement micro-ondes (21) et lesdites sources de signal de rayonnement ou micro-ondes à semi-conducteurs sont configurées lors de leur excitation pour générer et/ou libérer ledit champ de rayonnement micro-ondes sous-jacent (25) ou le signal sous-jacent (26).

6. Unité de chauffage par micro-ondes (1) selon la revendication 5, comprenant une unité de source d'alimentation (40) qui est configurée pour exciter l'unité de rayonnement micro-ondes sous-jacente (20) et en particulier la ou les plusieurs source(s) de signal de rayonnement ou de rayonnement micro-ondes (21) et qui comprend en particulier une ou plusieurs source(s) d'alimentation en courant continu CC (41).

7. Unité de chauffage par micro-ondes (1) selon l'une quelconque des revendications précédentes, dans laquelle
- ladite unité de rayonnement micro-ondes (20) et en particulier une source de signal de rayonnement ou de rayonnement micro-ondes (21) de celle-ci est ou comprend un amplificateur à transistors (103) et/ou
- ladite unité d'adaptation d'impédance (30) et en particulier un réseau d'adaptation d'impédance (101) de celle-ci comprend un port de rétroaction micro-ondes et/ou un port de rétroaction de signal micro-ondes qui est connecté à une entrée de l'amplificateur à transistors (103) afin de satisfaire à une condition d'oscillation sous-jacente.

8. Unité de chauffage par micro-ondes (1) selon la revendication 7, dans laquelle un dispositif de correction d'amplitude et/ou de phase intelligent supplémentaire (105) est ou sont connecté(s) entre le port de rétroaction et l'entrée de l'amplificateur à transistors (103).

9. Unité de chauffage par micro-ondes (1) selon l'une quelconque des revendications précédentes, dans laquelle
- l'unité de rayonnement micro-ondes (20) et en particulier une source de rayonnement micro-ondes (27) et/ou une source de signal de rayonnement micro-ondes (21) de celle-ci est ou comprend un amplificateur à transistors (103) et en particulier
- a une entrée qui est connectée à une sortie d'une source de signal de faible puissance (104) et/ou
- la source de signal de faible puissance (104) est ou comprend au moins l'un d'une boucle à verrouillage de phase (PLL), d'un oscillateur commandé en tension (VCO) et d'une synthèse numérique directe (DDS).

10. Unité de chauffage par micro-ondes (1) selon l'une quelconque des revendications précédentes, qui est formée dans ou comme au moins l'un d'un produit et/ou dispositif d'administration de médicament, d'un dispositif ou d'un produit d'inhalation (1'), d'un produit ou dispositif à fumer (1'), d'un produit ou dispositif mobile (1') et/ou d'un produit ou dispositif portable (1').

11. Unité de chauffage par micro-ondes (1) selon l'une quelconque des revendications précédentes, dans laquelle ledit oscillateur d'oscillations libres (109)
- est connecté à l'unité de maintien et d'exposition d'échantillon (10) ou fait partie de celle-ci, et/ou
- est, comprend et/ou réalise un modulateur IQ et/ou la fonctionnalité d'un modulateur IQ qui signifie en particulier la capacité de commander la phase et/ou le gain, et/ou
- comprend à l'intérieur d'une boucle de rétroaction (106) un ou l'amplificateur à transistors (103), une ou l'unité d'adaptation d'impédance (30) et/ou un réseau d'adaptation d'impédance (101) et un ou le dispositif de correction d'amplitude/phase (105).

12. Procédé de chauffage par micro-ondes et/ou RF pour chauffer un échantillon de formation d'aérosol (2) en raison de l'absorption des micro-ondes par un matériau (102) dudit échantillon (2) et pour libérer ainsi par ou à partir dudit échantillon (2) lors du chauffage dudit échantillon (2) au moins un aérosol, l'au moins un aérosol comprenant au moins une substance phyto-active et un principe actif pharmaceutique,
- dans lequel une unité de chauffage par micro-ondes (1) selon l'une quelconque des revendications précédentes est utilisée pour chauffer ledit échantillon (2) et
- dans lequel en particulier au moins l'un
- d'un substrat de formation d'aérosol (102) d'un article à fumer, en particulier d'un article à fumer contenant de la nicotine et/ou sans nicotine,
- d'un échantillon de formation d'aérosol solide chargé de tabac d'un échantillon à fumer, en particulier d'un échantillon à fumer contenant de la nicotine et/ou sans nicotine,
- d'un substrat de formation d'aérosol avec ou d'un liquide contenant de la nicotine et/ou sans nicotine,
- d'un principe actif pharmaceutique de formation d'aérosol,
- d'une substance phyto-active de formation d'aérosol et en particulier provenant de matière première végétale et/ou de matière végétale,
- du delta-9-Tétrahydrocanabinol (THC) et/ou du cannabidiol (CBD) et/ou des cannabinoïdes de formation d'aérosol contenant une matière végétale,
- d'un produit médicamenteux botanique et/ou d'une substance médicamenteuse botanique de formation d'aérosol,
- d'un substrat de formation d'aérosol (102) ayant au moins l'un parmi des sensibilisateurs et/ou des absorbeurs de micro-ondes du groupe de substances qui comprend des polysilsesquioxanes, des nanotubes de carbone, du graphite, du graphène, du carbone activé, du charbon actif, une poudre métallique, une poudre semi-conductrice fonctionnalisés et des combinaisons et des mélanges de ceux-ci,
est ou sont utilisé(s) et/ou chauffé(s) comme ledit échantillon (2) ou comme une partie de celui-ci.

13. Procédé de chauffage par micro-ondes selon la revendication 12, dans lequel une température de l'échantillon de formation d'aérosol (2) est mesurée ou/et approximée dont une valeur est fournie à une unité de boucle de commande de température commandant une puissance fournie pour stimuler un procédé de génération et/ou de libération de micro-ondes.

14. Procédé de chauffage par micro-ondes selon l'une quelconque des revendications 12 et 13, dans lequel :
- une fréquence de fonctionnement se trouve dans la plage allant d'environ 1 MHz à environ 15 GHZ, de préférence dans une bande ISM et/ou dans une plage d'environ 2,4 GHz à environ 2,5 GHz et/ou avec une fréquence centrale d'environ 2,45 GHz,
- une puissance de fonctionnement en mode continu se trouve dans une plage allant d'environ 0,1 mW à 50 Watt et de préférence autour d'environ 2 W,
- une puissance de fonctionnement en mode pulsé se trouve dans une plage allant d'environ 0,1 mW à environ 50 W et de préférence autour d'environ 3 W,
- une puissance de fonctionnement est augmentée entre une valeur d'environ 0,1 Wet une valeur d'environ 30 W, et/ou
- une fréquence de fonctionnement est réglée sur une valeur constante,
- une fréquence de fonctionnement varie avec le temps et en particulier balayée entre une valeur minimale et la valeur maximale, en particulier entre environ 2,4 GHz et environ 2,5 GHz.

15. Procédé de chauffage par micro-ondes selon l'une quelconque des revendications 12 à 14, dans lequel un échantillon de formation d'aérosol (2) est utilisé, qui :
- est formé comme, comprend, est contenu à l'intérieur et/ou est emballé à l'intérieur d'au moins l'un d'un matériau fluide, d'un matériau solide, d'un comprimé, d'une capsule, d'une cartouche, d'un flacon à échantillon cylindrique, d'une pastille et/ou peut être adapté selon la taille d'une cavité sous-jacente (16) et/ou des excipients pharmaceutiques sont ajoutés à l'échantillon de formation d'aérosol (2), et/ou
- est ou forme un récipient et/ou a un boîtier, dans lequel le boîtier est en particulier transparent vis-à-vis du rayonnement micro-ondes, comprend ou est formé en plastique, en matériau synthétique, en polypropylène, en verre, en quartz et/ou a un faible facteur de perte diélectrique comparable (ε") et/ou une faible tangente d'angle de pertes comparable (tan(δ)), et/ou
- est contenu dans un récipient formé en tant qu'embout (119) qui est configuré pour permettre l'inhalation de l'aérosol formé, en tant qu'une seule pièce, en tant qu'élément d'élimination et/ou pour comprendre un filtre (115), en particulier à efficacité de filtration de particules faible ou très faible, et/ou un tube creux.
